# EUROPEAN PATENT SPECIFICATION

(11) **EP 3 319 469 B1**
(45) Date of publication and mention of the grant of the patent: **16.09.2020**
(21) Application number: 16821771.9
(22) Date of filing: 26.05.2016
(51) Int. Cl.: A43B 7/14, A61F 5/052, A61F 5/14, A43B 17/00, A43B 13/02, A61F 5/01, A43B 7/20

(54) **FORCE DELIVERY IN ORTHOTIC, ORTHOTIC INSERTS AND ANKLE FOOT ORTHOSIS PRODUCTS AND SYSTEMS**
KRAFTABGABE IN DER ORTHETIK, ORTHETISCHE EINLAGEN UND FUSSGELENKORTHESENPRODUKTE UND SYSTEME
LIBÉRATION DE FORCE EN ORTHÉTIQUE, INSERTS ORTHÉTIQUES ET PRODUITS ET SYSTÈMES D'ORTHÈSE CHEVILLE-PIED

(30) Priority: 07.07.2015 US 201562189400 P
(43) Date of publication of application: 16.05.2018
(73) Proprietor: Roar Athletic Performance Corp., Milford, CT 06461 (US)
(72) Inventor: ARCIUOLO, Matthew J., Milford, CT 06461 (US)
(74) Representative: Studio Torta S.p.A.
(86) International application number: PCT/US2016/034373
(87) International publication number: WO 2017/007536

(56) References cited:
- EP-A1- 2 524 674
- WO-A2-2015/052585
- US-A- 4 520 581
- US-A- 4 520 581
- US-A1- 2004 134 500
- US-A1- 2004 134 500
- US-A1- 2005 054 959
- US-A1- 2005 054 959
- US-A1- 2007 234 592

## Description

### BACKGROUND

### 1. TECHNICAL FIELD

The present disclosure generally relates to orthotics, orthotic inserts and ankle foot orthoses and, in particular, to force delivery systems and substrates for use in fabricating orthotics, orthotic inserts and ankle foot orthosis products and systems that function, *inter alia,* to increase propulsion.

### 2. BACKGROUND ART

Foot orthotics are often used to compensate for impaired foot function by controlling abnormal motion across the joints of the foot. Specific impairments that a foot and/or ankle-foot orthotic (AFO) may assist include mild "foot drop" due to neurological conditions, orthopedic gait abnormality, clubfoot, mid-tarsal fracture, partial foot amputation, arthritis, hallux valgus, hallux rigidus, turf toe, and plantar fasciitis. Foot orthotics and/or ankle-foot orthotics may also be prescribed and/or employed to reduce pain, to provide support, to prevent foot deformity and/or to prevent the worsening thereof, to relieve pressure on a certain area of the foot, and/or to improve the overall biomechanical function of the foot and lower extremity limbs.

Foot orthotics normally include a specially fitted insert or footbed for use in conjunction with a shoe. Foot orthotics may provide support for the foot by distributing pressure or realigning foot joints while standing, walking or running. As such, foot orthotics are often used by athletes to relieve symptoms associated with a variety of soft tissue inflammatory conditions, e.g., plantar fasciitis. Also, foot orthotics have been designed and/or used to address arch support or cushioning requirements.

According to the 2005 Americans with Disabilities report, approximately 27 million people over the age of 15 had a walking-related disability. Ankle joint musculature plays an important role during walking and is thought to be the primary muscle group that supports upright stance and produces forward propulsion. Individuals with muscular weakness about the ankle, an impairment often caused by upper motor neuron disorders and lower extremity injuries, are frequently prescribed ankle-foot orthoses which brace the ankle during gait and aim to improve gait function.

Generally, foot orthotics are designed to remove pressure and/or stress from painful areas of the foot and ankle. The main focus of orthotic technology has been to increase the comfort and cushioning of the product. Shock attenuation (absorption) has been addressed by myriad footwear innovations in the past, but efforts at increasing the efficiency of motion have been largely absent. Foot orthotics may also function to address positioning and movement of the foot, ideally addressing balance issues. Many foot orthotics deliver an equal or constant stiffness along their length which can contribute to gait and/or balance issues that the foot orthotic is intended to improve and/or resolve.

Beyond the realm of foot orthotics, ankle-foot orthosis have been developed that are intended to substitute and/or compensate for various anatomical issues, e.g., weak dorsiflexors during the swing phase and weak plantarflexors during the stance phase of a user's gait. In general, ankle-foot orthosis systems may function to support and align the ankle and the foot and generally improve the functions of the foot with particular focus on ankle/knee biomechanics.

The products that are currently on the market in this category are generally designed to assist the impaired individual in gaiting more normally. The focus of prior designs in the orthotic/prosthetic marketplace has been to substitute, with a mechanical device, the normal operation of the human foot/ankle/leg complex. Consistent with this focus, improvements in the orthotic/prosthetic marketplace have been aimed at replacing the normal function of the impaired lower extremity complex. However, in addition to assisting such individuals to gait more normally, it is desirable to also improve the ability of the impaired individual to propel themselves forward.

US 2005/0054959 discloses an orthotic footplate having a plurality of fiber layers of varying lengths. The fibers of a first layer adjacent to a ground surface are oriented at an oblique angle with respect to a line of progression of the footplate extending from a heel portion to a middle portion of a toe portion of the footplate.

Despite efforts to date, there remains a need for improved force delivery systems and substrates for use in fabricating orthotics, orthotic inserts and ankle foot orthosis products and systems that function, *inter alia,* to improve biomechanical function, including biomechanical function of the foot, ankle and/or knee. Furthermore, a need remains for orthotics, orthotic inserts and ankle-foot orthosis products/systems that impart effective and efficient propulsive force in connection with a user's gait. Still further, a need exists for products/systems that function to assist or improve the ability of the human foot/leg complex of an impaired individual to spring or propel the individual either forward or upward (or any combination of the two).

### SUMMARY OF THE DISCLOSURE

The invention is defined by the independent claim 1. Preferred embodiments are the subject of the dependent claims. The present disclosure advantageously meets the needs of end users interested in improving the efficiency of motion in relation to normal activity. Instead of just attempting to replace lost function, the products, systems and methods of the present disclosure increase the amount and rate of plantarflexion to assist in gait. Thus, the products, systems and methods of the present disclosure, in addition to assisting able-bodied individuals, also may be used to assist individuals who suffer from an array of neurological and/or physical impairments.

The present disclosure provides an advantageous flexible member that delivers a desirable force profile when employed as an orthotic, orthotic insert and/or ankle foot orthosis (AFO). The flexible member is generally configured and dimensioned to cooperate with and correspond to the shape/geometry of a human foot and improves biomechanical function, including biomechanical function of a foot, ankle and/or knee. The disclosed flexible member advantageously imparts propulsive force in connection with a user's gait by storing and releasing an individual's own energy to assist in walking and/or standing. In particular, the flexible member functions, *inter alia,* to increase and/or maximize propulsion at push off.

In exemplary implementations, the disclosed flexible member is fabricated, at least in part, from a plurality of fibers that are generally aligned, i.e., parallel, with respect to each other and are oriented relative to the axis of the flexible member at a predefined angle, e.g., angled lateral-to-medial at approximately 15° relative to an axis that runs from heel center to toe center. In fabricating the disclosed flexible member, the carbon fibers may be incorporated into fabric sheets, e.g., using one or more resins, and the fabric sheets may then be layered to deliver a desired force-response functionality.

In exemplary embodiments, the disclosed fibers may take the form of pre-impregnated ("pre-preg") composite fibers in which a matrix material, such as an epoxy resin, is already present. The fibers are un-idirectionally aligned and the matrix advantageously functions to bond them together in a fixed orientation relative to each other. In fabricating the flexible member of the present disclosure, multiple pre-preg sheets are stacked with a desired alignment of the fibers themselves (layer-to-layer), and a molding operation is initiated that delivers heat to the pre-preg sheets to cure them in the desired orientation.

The disclosed flexible members are advantageously designed and fabricated with varying amounts of resistance or spring at specific parts thereof. Thus, when employed as an orthotic, orthotic insert and/or AFO, the disclosed fiber layers are advantageously arranged such that the flexible member delivers a desired level of stiffness where the user needs/desires it to be stiff and a desired level of flexibility where such flexibility is necessary/desirable. Of note, orthotics are customarily shaped to mirror the shape and motion of the foot. Orthotics that employ the disclosed flexible members, in distinct contrast, are generally shaped in the opposite direction, thereby using the body's own weight to load a spring force associated with the disclosed flexible member, and thereafter, the user's own motion translates to an increase in the spring potential of the orthotic. Based on the stiffness and design criteria associated with the disclosed fiber-based flexible member, the spring force is advantageously unloaded at a rapid rate, propelling the user forward.

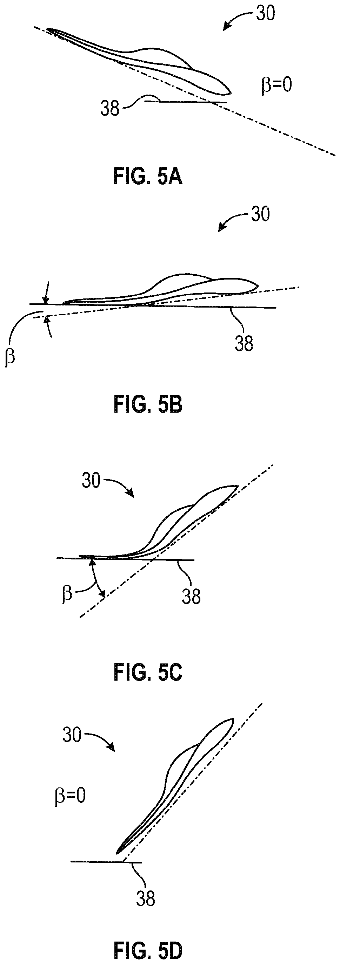

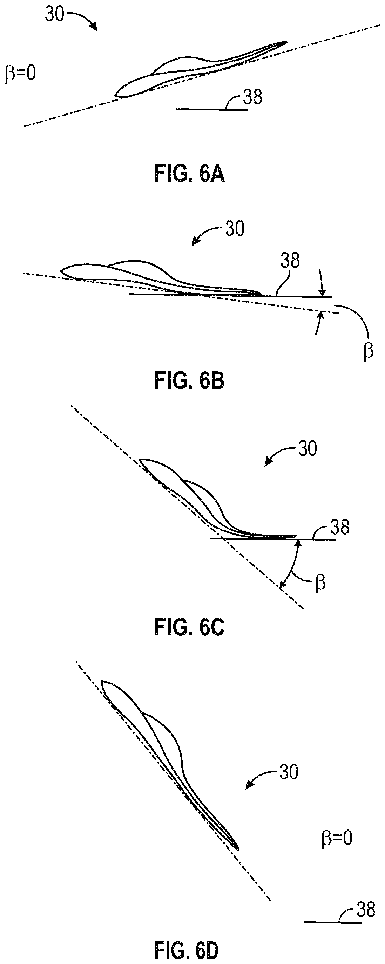

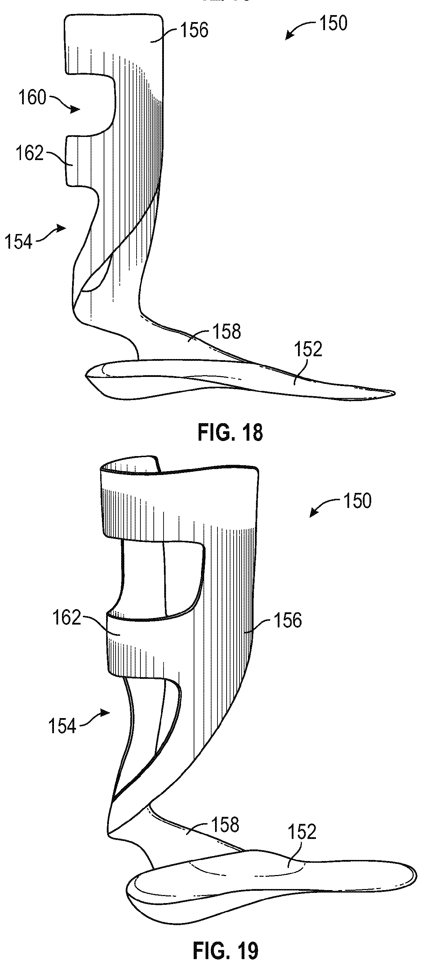

Of note, there are four (4) phases of gait. The disclosed flexible member, e.g., when employed in connection with an orthotic, orthotic insert and/or AFO product/system advantageously enhances propulsion across the four phases of gait, as described hereinbelow:
- Heel strike: When the foot initially contacts the ground while walking or running. At heel strike, the posterior (rear) of the flexible member deflects slightly, attenuating shock, storing energy and allowing a smooth flow to the next phase.
- Foot Flat (Stance Phase): When both the heel and the forefoot are on the ground at the same time. At foot flat, the flexible member's slight arch from heel to toe provides a pre-load to increase the spring force going into the next gait phase (see, e.g., the downward force represented by Arrow "X" in Fig. 18 that establishes the noted pre-load in an exemplary AFO implementation). A secondary benefit to the arched shape of the flexible member from heel-to-toe is that when the flexible member deflects, the posterior strut associated with the exemplary AFO implementation moves forward, providing added "push" during gait (see, e.g., the forward force represented by Arrow "Y" in Fig. 18).
- Heel off: When the foot is dorsiflexed with the heel off of the ground (see, e.g., Figs. 5B/5C and Figs. 6B/6C). At heel off, when the foot is maximally flexed is when the potential energy of the flexible member is stored, ready to be released.
- Toe off: When the foot leaves the ground on its way to the next phase. At toe off is when the potential energy stored in the "stance phase" and "heel off' phases of gait is released, increasing the force and rate of plantarflexion, propelling the user forward (and/or upward). This force delivery may be utilized in numerous applications and environments, e.g., to assist an impaired individual in walking and/or assist an athlete in performing/competing.

In exemplary implementations of the present disclosure, the flexible member is fabricated, in whole or in part, from pre-impregnated carbon fiber composite. Of note, pre-impregnated carbon fiber composites may be used to deliver desired levels of stiffness and flex in a precise manner through placement so that maximum (and/or desired) spring force can be achieved to assist propulsion of the impaired individual. The flexible member may be employed independently, e.g., as an orthotic or as an orthotic insert, or may be attached/connected to an ankle/leg brace structure to provide lower leg bracing. The attachment/connection may be permanent or designed to facilitate detachment therebetween. The orthotic, orthotic insert and/or AFO may be advantageously inserted into appropriate footwear, and may function to assist an individual who is suffering from various maladies and/or pathologies, e.g., to compensate for muscle weakness (foot drop) caused by stroke, spinal cord injury, muscular dystrophy, cerebral palsy, peripheral neuropathy and less commonly, polio amongst other conditions.

The carbon fiber composites may be advantageously arrayed in layers to deliver desired force response characteristics. Moreover, the fiber alignment may be selected so as to deliver a desired force response. Thus, in exemplary implementations of the present disclosure, a plurality of carbon fiber layers are arranged so that the flexible member is the stiffest where the pressure is greatest and gradually exhibits greater flexibility (i.e., less rigidity) as it extends distally toward the toe region. As noted in the gait-related discussion above, a purpose of the flexible member is to pre-load a spring force at the heel off phase of the human gait cycle, and then to unload the pre-loaded spring force upon toe-off. Since the pre-loaded spring force cannot move the ground beneath the user, it necessarily and advantageously moves the user. More particularly, the loaded spring force releases its potential energy as the user picks his/her foot up off of the ground on the way to the next step. As such, the flexible member increases the plantarflexion moment (rate of downforce) as the bottom of the metatarsal heads distally to the toe region, propelling the user forward and/or upward, depending upon the applicable user activity.

According to another exemplary embodiment of the present disclosure, the flexible member may be incorporated into a foot ankle orthotic that includes a footplate formed, in whole or in part from the flexible member, and a brace structure. The footplate may include a toe platform, the toe platform comprising a toe, sulcus, and ball; a longitudinal arch pad in communication with the toe platform; a heel cup in communication with the longitudinal arch pad, the heel cup comprising a heel; where in order to form an angle β that is greater than 0° between the toe platform and the remainder of the orthotic, a pre-load pressure P is required. The brace structure is joined with respect to the footplate and is configured and dimensioned for securement with respect to the lower leg region of a user. The brace structure may be secured with respect to the user's leg from the front, back, side and/or a combination thereof. Thus, the securement mechanism may be accessed from an anterior, posterior, medial and/or lateral direction relative to the patient's leg.

These, and other aspects and objects of the present disclosure will be better appreciated and understood when considered in conjunction with the following detailed description and accompanying drawings.

### BRIEF DESCRIPTION OF THE DRAWINGS

The features, aspects, and advantages of the present disclosure, as detailed in the following description, will be better understood by reference to the accompanying drawings, in which:
**FIG. 1** is a top view of an exemplary flexible member for a right foot according to the present disclosure;
**FIG. 2** is a cross-sectional side view of the exemplary flexible member of FIG. 1 taken along line 2-2;
**FIG. 3** is a perspective top view of the exemplary flexible member of FIG. 1;
**FIGS. 4A** and **4B** are side and top perspective views of a footplate for a right foot, respectively, according to an exemplary embodiment of the present disclosure;
**FIGS. 4C** and **4D** are front and side perspective views of an orthotic footplate for a left foot, respectively, according to another exemplary embodiment of the present disclosure;
**FIGS. 5A-D** are schematic views of the right orthotic footplate at various phases of human gait according to an exemplary embodiment of the present disclosure;
**FIGS. 6A-D** are schematic views of the left orthotic footplate at various phases of human gait according to an exemplary embodiment of the present disclosure;
**FIG. 7** is a side perspective view of the lateral side of a right orthotic footplate according to another exemplary embodiment of the present disclosure;
**FIG. 8** is a perspective view of an ankle foot orthosis (AFO) for use on the lower right limb of a patient according to an exemplary embodiment of the present disclosure;
**FIG. 9** is a perspective view of the medial side of the AFO of FIG. 8 according to an exemplary embodiment of the present disclosure;
**FIG. 10** is a front perspective view of the AFO of FIG. 8 according to an exemplary embodiment of the present disclosure;
**FIG. 11** is a back perspective view of the AFO of FIG. 8 according to an exemplary embodiment of the present disclosure;
**FIG. 12** is a side view of the AFO of FIG. 8 according to an exemplary embodiment of the present disclosure;
**FIG. 13** is a perspective view of the side of the AFO of FIG. 8 according to an exemplary embodiment of the present disclosure;
**FIG. 14** is a perspective view of the AFO of FIG. 8 according to an exemplary embodiment of the present disclosure;
**FIG. 15** is a perspective view of the medial side of the AFO of FIG. 8 according to an exemplary embodiment of the present disclosure;
**FIG. 16** is a front view of an alternative AFO according to an exemplary embodiment of the present disclosure;
**FIG. 17** is a rear view of the alternative AFO of FIG. 16 according to an exemplary embodiment of the present disclosure.
**FIG. 18** is a side view of the alternative AFO of FIG. 16 according to an exemplary embodiment of the present disclosure;
**FIG. 19** is a perspective view of the side of the AFO of FIG. 16 according to an exemplary embodiment of the present disclosure;
**FIGS. 20** and **21** are front and rear views of an alternative AFO according to the present disclosure;
**FIGS. 22-24** are side views of alternative AFO's according to the present disclosure.

### DESCRIPTION OF EXEMPLARY EMBODIMENTS

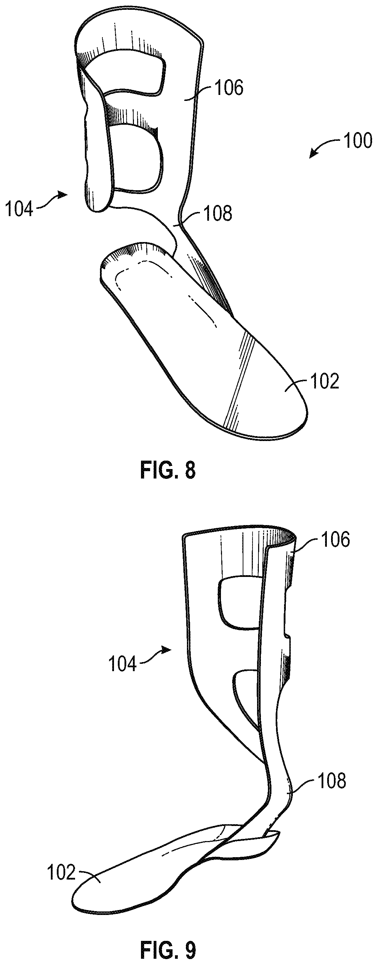

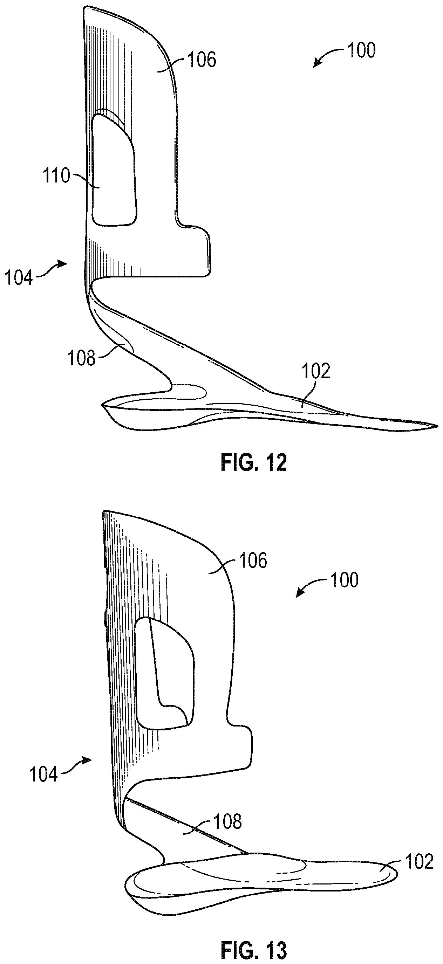

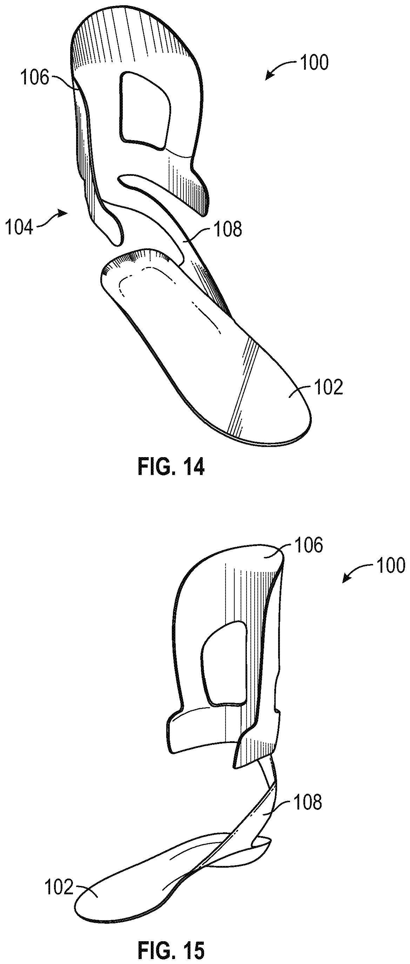

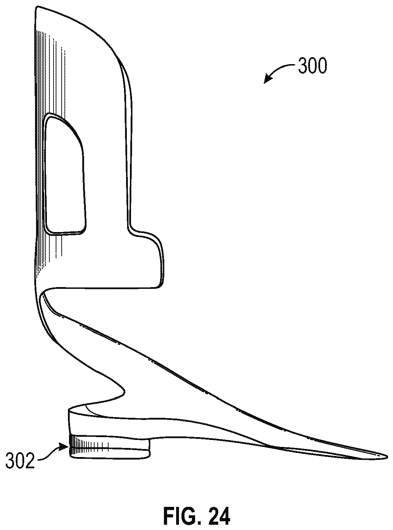

The following description details exemplary flexible members for use in orthotics, orthotic inserts and ankle foot orthosis products/systems according to the present disclosure. Of note, several of the figures, e.g., FIGS. 8-24, relate to exemplary implementations of the disclosed flexible members in connection with AFO products/systems. However, as will be readily apparent to persons skilled in the art, the present disclosure is not limited by or to the exemplary embodiments disclosed herein, including specifically the exemplary AFO products/systems described with reference to FIGS. 8-24, but extends to and encompasses variations and/or modifications that draw upon the innovative products, systems and modalities described herein, including specifically orthotic and orthotic insert applications and implementations.

The disclosed flexible member enables use of a person's own energy and returns it to the individual, thereby advantageously increasing the downforce exerted during walking or running upon the ground. The overall force profile of the disclosed flexible member thus functions to propel the user forward or upward, whichever is desired. The disclosed flexible member design also increases the dorsiflexion moment, thereby assisting the individual in clearing the ground during swing phase in order to advance to heel strike efficiently and effectively. This increase in propulsive capability is invaluable for individuals suffering from neurological impairment resulting in impaired dorsiflexion control or "foot drop." Indeed, the additional energy return provided by the disclosed flexible member during plantarflexion functions to replace (or augment) the propulsion that a normal foot-ankle complex would generate, thereby improving balance, forward movement and proprioception in an individual.

The disclosed flexible member is designed to increase propulsivity in walking, running and jumping activities. The flexible member is generally designed with about a 15° plantar flexion from the ball of the foot to the toe, and about a 5° plantar flexion from the 5th metatarsal to the hallux. Based on the noted design, as the user progresses through the phases of gait, the flexible member progressively loads potential energy at "foot flat" and "heel-off', and releases that energy at "toe off'.

The flexible member may be advantageously fabricated using "pre-impregnated" or "pre-preg" composite fibers where a material, such as epoxy, is already present. The pre-preg composite fibers, e.g., carbon fibers, are uni-directionally aligned at an angle relative to an axis that extends from the heel center to the toe center (the "center axis"). The fibers are angled at an angle of between about 10° and 20° relative to the center axis such that the fibers are angled medially from heel-to-toe. In further exemplary embodiments, the fibers are angled at an angle of between about 12° and 18° relative to the center axis such that the fibers are angled medially from heel-to-toe, and preferably the fibers are angled at about 15° relative to the center axis such that the fibers are angled medially from heel-to-toe.

The medially angled orientation of the unidirectionally aligned fibers is biomechanically advantageous because normal human step patterns run in that direction. More particularly, when a heel contacts the ground as an individual starts his/her step, the heel generally contacts the ground on the outside of the heel, i.e., laterally. As the step progresses, the foot of the individual ultimately leaves the ground in the region of the big toe, i.e., medially. Consistent with the typical step progression noted herein, the unidirectional fibers are generally angled medially-to-laterally as they run from heel-to-toe, thereby roughly aligning with the step progression and optimizing the performance of the disclosed flexible member when employed as an orthotic, orthotic insert or as part of an AFO product/system.

The pre-preg composite fibers typically contain an amount of matrix material used to bond them together and to other components during manufacture. In exemplary embodiments, the matrix material may be an epoxy resin, e.g., bisphenol A and/or bisphenol F epoxy resins. The pre-preg composite fibers are generally stored in cooled areas, since activation is most commonly done by heat.

In exemplary implementations of the present disclosure, unidirectionally aligned pre-preg carbon fibers are employed in fabricating the disclosed flexible member. Owing to the use of "pre-preg" carbon fiber in the disclosed flexible member, the flexible member can be designed with varying amounts of resistance or spring at specific parts and/or regions of the flexible member. Depending on how the pre-preg carbon fiber layers are arranged, the flexible member can be stiff where the user needs it to be stiff, and flexible where desired and/or required. Pre-preg layering offers superior flexibility and results as compared to standard carbon fiber in that it can be tailored to accomplish an increase in propulsion by increasing the natural spring effect of the human arch and foot structure in a flexible member. The carbon fiber layers may be thickest under the ball of the foot and to the heel where the weight is the greatest and gradually get thinner distally under the user's toe region.

This layering process tailors the spring effect of the flexible member so that it is stiff where it is needed and flexible where it is necessary to maximize its effect on the human foot. Of note, orthotics are customarily shaped to mirror the shape and motion of the foot. The disclosed footplate is generally shaped in the opposite direction, thereby using the body's own weight to load spring force into the flexible member, and further using the user's own motion to increase spring potential. Owing to the stiffness and lightweight characteristics of carbon fiber, the pre-loaded spring force is advantageously unloaded at a rapid rate, propelling the user forward.

The disclosed flexible member design loads a spring force while the user is simply standing still and this spring effect is amplified when the toes are dorsiflexed (turned up). As the foot leaves the ground, preparing for its next heel strike, the flexible member unloads into plantarflexion at a rapid rate using ground reactive force to propel the user forward by amplifying push-off.

The disclosed flexible member may be made from pre-preg carbon fiber fabrics, although alternative fiber materials may be employed (in whole or in part), e.g., glass fibers, aramid fibers and the like. The carbon fiber fabric may be shipped as a dry loosely woven cloth. A variety of methods may be used to apply wet epoxy resin to the cloth. After application of the epoxy resin, the cloth/resin combination generally cure at room temperature. In forming the disclosed flexible member, a molding operation is generally employed. The pre-preg carbon fibers/woven cloth may be applied in layers to an appropriately sized/configured mold. Once positioned within the mold, a clear plastic sheet may be mounted over the pre-preg fibers/cloth and affixed to the edges of the mold, e.g., with foam tape, thereby creating an air tight seal between the inside of the mold and the outside. A vacuum pump is then used to apply a vacuum within the mold as air is removed. As the air is removed, the plastic presses against the pre-preg fibers/cloth and against the inside of the mold. The pre-preg is allowed to cure within the mold as heat is applied to the fiber/mold. The thermoset resin (e.g., bisphenol A and/or bisphenol F) cures at an elevated temperature, undergoing a chemical reaction that transforms the pre-preg into a solid material that is highly durable, temperature resistant, exceptionally resilient and extremely lightweight. Thereafter, the cured fiber system is separated from the mold.

The carbon fiber layers are generally placed in such a way that there are more layers under the metatarsal heads (ball of the foot), where there is the most downforce exerted by the foot, gradually getting thinner (less layers) progressively approaching the toe region, where there is less downforce. This ability to gradually lower the stiffness of the flexible member moving distally from heel-to-toe delivers maximum spring force to the user.

To maximize the spring effect, the flexible member is shaped in a slight arc from heel-to-toe so that just by the user stepping on the flexible member, a slight pre-load is achieved. The flexible member is also slightly torqued so that the medial distal aspect (under the great toe) is lower than the lateral aspect (little toe). This torqued/arched geometric arrangement maximizes the spring effect by using the natural flow of the gait cycle, which generally runs laterally from the heel to medially at the great toe. Moreover, the arched shape allows the flexible member to deflect plantarly. Thus, in the case of an AFO product/system, the rear of the brace structure of the AFO may be caused to push the leg forward in the calf region, thereby allowing easier/more effective propulsion.

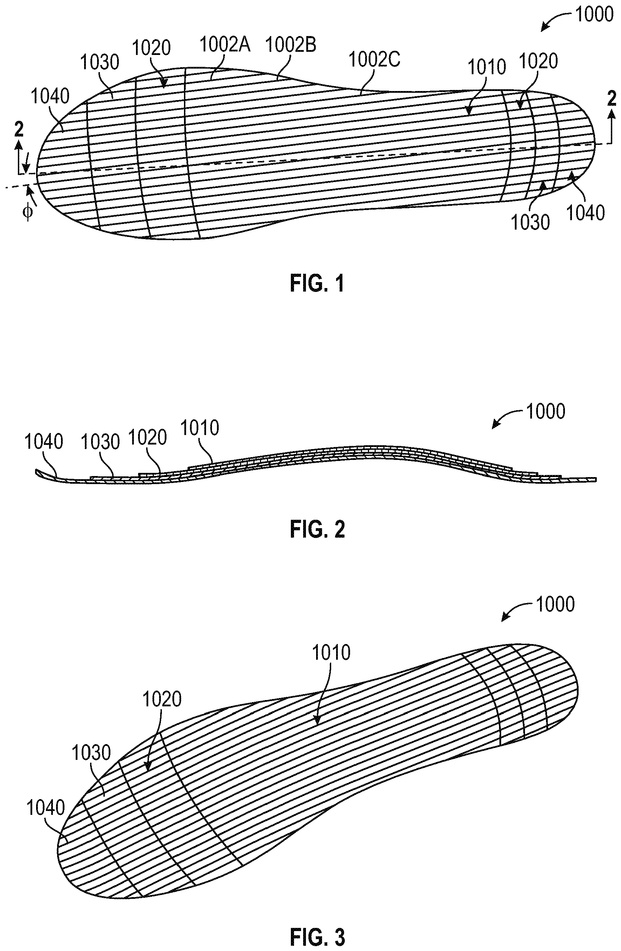

With initial reference to FIGS. 1-3, an exemplary flexible member 1000 according to the present disclosure is schematically depicted. As shown in FIGS. 1 and 3, a plurality of unidirectionally aligned fibers 1002A, 1002B, 1002C extend generally from heel-to-toe. However, with specific reference to FIG. 1, the line 2-2 defines a central axis for the disclosed flexible member 1000 in that it extends from the center of the heel region to the center of the toe region. As is apparent from FIG. 1, an angle φ is defined between the center axis defined by the line 2-2 and the unidirectional fibers. The angle φ is between about 10° and 20° relative to the center axis such that the fibers are angled medially from heel-to-toe, preferably between about 12° and 18° relative to the center axis such that the fibers are angled medially from heel-to-toe, and more preferably about 15° relative to the center axis such that the fibers are angled medially from heel-to-toe.

The number of unidirectional fibers incorporated into the disclosed flexible member 1000 is generally selected to achieve the desired force-response behavior. However, as shown in the cross-sectional view of FIG. 2, exemplary implementations of the disclosed flexible member 1000 include a plurality of layers of fibers. In exemplary implementations of the present disclosure, the fibers in each layer are unidirectionally aligned and are angled relative to the center axis in the same, or very closely similar, levels, e.g., between about 10° and 20° relative to the center axis, preferably between about 12° and 18° relative to the center axis, and more preferably about 15° relative to the center axis.

With further reference to FIG. 2, an exemplary implementation of the present disclosure may include four (4) fiber layers 1010, 1020, 1030, 1040. The top-most layer 1010 is the shortest layer, whereas the bottom-most layer 1040 is the longest layer. The intermediate layers 1020, 1030 have a greater length extent as compared to top-most layer 1010, but a shorter length extent as compared to bottom-most layer 1040. In the central region of flexible member 1000, where all four layers are present in the cross-section of FIG. 2, the greatest stiffness/rigidity is imparted to flexible member 1000. As the layers "thin", i.e., in the regions closer to the heel and to the toe of the flexible member 1000, greater flexibility is imparted to flexible member 1000. The transitions from thicker to thinner cross-section are generally selected to deliver the desired force response/flexibility, e.g., as described with reference to FIGS. 5 and 6 below, and are almost imperceptible to users of the disclosed flexible members.

As is apparent from the schematic depictions of FIGS. 1-3, exemplary implementations of the fiber-based flexible members of the present disclosure are characterized in part by the following parameters:
- Unidirectionally aligned fibers;
- Angled orientation of the aligned fibers relative to the "center axis" of the flexible member, e.g., between about 10° and 20° relative to the center axis (and preferably about 15° relative to the center axis);
- Multiple fiber layers of varying lengths;
- Greater thickness in the central region as compared to front/toe and back/heel regions; and
- Selection of number of fibers, number of layers and relative lengths of layers based on desired force-response and flexibility/rigidity factors.

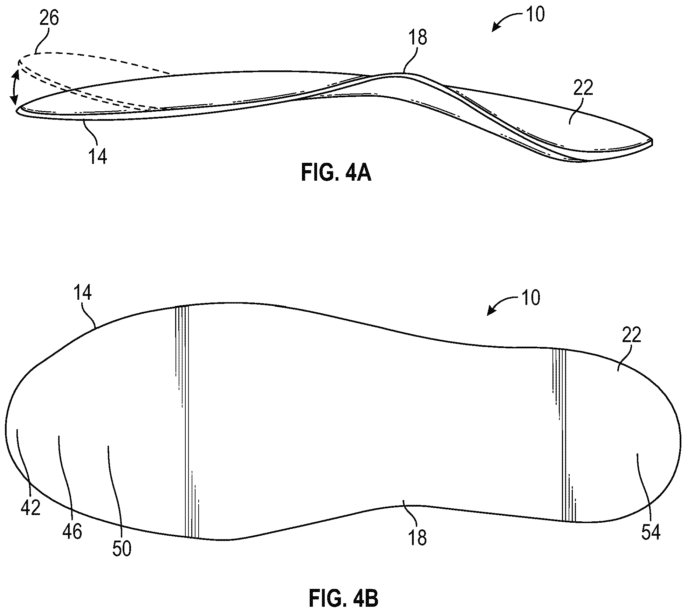

With further reference to the appended figures, reference is made to FIGS. 4A-4D and FIGS. 5-7 which relate specifically to a footplate design that incorporates the flexible member according to exemplary embodiments of the present disclosure. In particular, FIG. 4A is a side view of one embodiment of an exemplary footplate 10 according to the present disclosure. This figure shows a right foot footplate. One of ordinary skill will recognize that the present disclosure also encompasses left foot footplates. The footplate 10 may have a toe platform 14, a longitudinal arch pad 18 and a heel cup 22. One embodiment of how the footplate 10 can preload the spring function of the footplate is shown in dashed line 26. The dashed line 26 shows how the toe platform 14 can flex with respect to the rest of the footplate, providing a preload in the footplate 10. When this preload is released, the footplate 10 may provide thrust or propulsion to the user.

FIG. 4B is a top view of the footplate 10 from FIG. 4A. FIG. 4B shows where thickness measurements were made below. Thicknesses were measured generally at the toe 42, sulcus 46, ball 50, and heel 54.

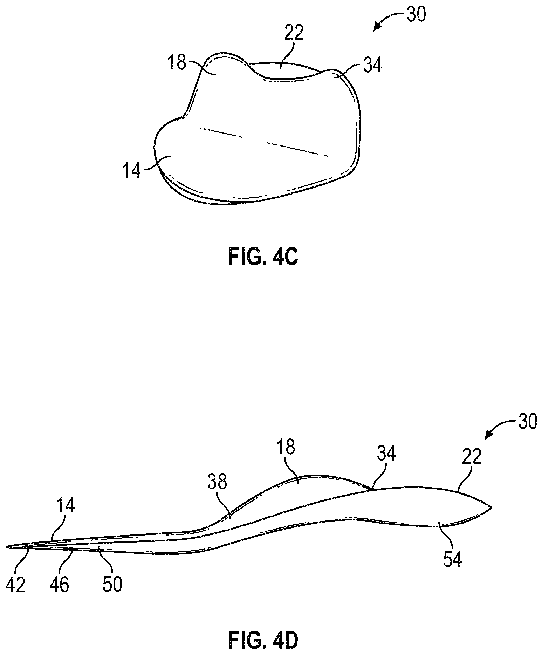

FIG. 4C is a generally front perspective view of another embodiment of the disclosed footplate 30. The shown footplate 30 is for a left foot. This embodiment of the footplate 30 may have a toe platform 14, a longitudinal arch pad 18, a heel cup 22, and a peroneal arch pad 34.

FIG. 4D is a side view of the footplate 30 from FIG. 4C. The thickness of the material that makes up the footplate 30 may vary. For instance, for a female small sized footplate, the thickness may be about 1 mm at the toe 42, about 1.25 mm at the sulcus 46, and about 1.5 mm at the ball 50 to the heel 54. The small sized female footplate may correspond to a ladies' shoe sizes 5-6. For a female medium sized footplate, the thickness may be about 1.25 mm at the toe 42, about 1.5 mm at the sulcus 46, and about 1.75 mm at the ball 50 to the heel 54. The medium sized female footplate may correspond to a ladies' shoe sizes 7-8. For a female large sized footplate, the thickness may be about 1.5 mm at the toe 42, about 1.75 mm at the sulcus 46, and about 2 mm at the ball 50 to the heel 54. The large sized female footplate may correspond to a ladies' shoe sizes 9-10. For a female extra-large sized footplate, the thickness may be about 1.75 mm at the toe 42, about 1.75 mm at the sulcus 46, and about 2.25 mm at the ball 50 to the heel 54. The extra-large sized female footplate may correspond to a ladies' shoe sizes 11-12.

For a male small sized footplate, the thickness may be about 1 mm at the toe 42, about 1.25 mm at the sulcus 46, and about 1.5 mm at the ball 50 to the heel 54. The small sized male footplate may correspond to men's shoe sizes 6-7. For a male medium sized footplate, the thickness may be about 1.25 mm at the toe 42, about 1.5 mm at the sulcus 46, and about 1.75 mm at the ball 50 to the heel 54. The medium sized male footplate may correspond to men's shoe sizes 8-9. For a male large sized footplate, the thickness may be about 1.5 mm at the toe 42, about 1.75 mm at the sulcus 46, and about 2 mm at the ball 50 to the heel 54. The large sized male footplate may correspond to men's shoe sizes 10-11. For a male extra-large sized footplate, the thickness may be about 1.75 mm at the toe 42, about 1.75 mm at the sulcus 46, and about 2.25 mm at the ball 50 to the heel 54. The extra-large sized male footplate may correspond to men's shoe sizes 12-13. Of course, one of ordinary skill in the art will recognize that smaller and larger thicknesses may be used depending on the amount of "spring effect" one desires from the disclosed footplate.

FIG. 5 shows the footplate 30 of a right foot during the different phases of a step or stride. FIG. 5-A shows the footplate 30 as the foot is about to strike the ground 38 heel first. At FIG. 5-A, the flex angle β is generally 0°, that is the angle made between the toe platform and rest of the footplate due to a force applied by a user to the footplate, generally during walking, running, and/or jumping. FIG. 5-B shows the footplate as the foot begins to leave the ground and a pre-load has already started to occur in the toe platform 14, such that angle β is about 20°. FIG. 5-C shows an even greater pre-load in the toe platform 14, such that there is an angle β of about 45°. FIG. 5-D shows the foot off of the ground 38, and the footplate 30 has expended its pre-load by providing thrust or propulsion to the user's foot and/or leg. The angle β is now back to 0°.

FIG. 6 shows the footplate 30 of a left foot during the different phases of a step or stride. FIG. 6-A shows the footplate 30 as the foot is about to strike the ground 38 heel first. At FIG. 6-A, the flex angle β between the toe platform 14 and the rest of the footplate 30 is generally 0° (or no angle). FIG. 6-B shows the orthotic as the foot begins to leave the ground and a pre-load has already started to occur in the toe platform 14, such that β is about 20°. FIG. 6-C shows an even greater pre-load in the toe platform 14, such that there is an angle β of about 45°. FIG. 6-D shows the foot off of the ground 38, and the footplate 30 has expended its pre-load by providing thrust or propulsion to the user's foot and/or leg. The angle β is now back to 0°.

In order to form a non-zero angle β, a pre-load force of F is required to create the pre-load (and the flex angle β). The force of course is spread over an area of the footplate, and in the table below will be described generally as a pressure (psi). The pressure required to create the flex angle β may range from about 1 psi (1 psi ≈ 6895 N·m⁻²) to about 100 psi. According to an exemplary embodiment of the disclosed footplate, the pressures P for various flex angles β are shown below:

| Flex Angle β | Pressure P |
|---|---|
| 10° | 6.7 psi |
| 20° | 9.4 psi |
| 30° | 12.8 psi |
| 40° | 16.8 psi |
| 50° | 23.8 psi |
| 60° | 28.3 psi |
| 70° | 32.8 psi |
| 80° | 37.2 psi |
| 90° | 39.5 psi |

One of ordinary skill in the art will recognize that the pressure associated with the flex angle β may be changed from the table above depending on the amount of "spring effect" one desires from the footplate.

The footplate 10, 30 works in that it decreases the rate of dorsiflexion of the toes (loading a spring) and increases the rate of plantarflexion of the toes (releasing the spring) in the 4^{th} phase of gait (e.g., FIGS. 5-D and 6-D). This phenomenon maximizes the first ray leverage against ground reactive forces, thereby imparting maximum force to improve propulsion linearly (forward) and vertically (up) and laterally (side to side).

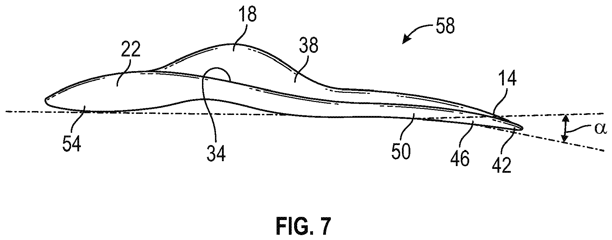

FIG. 7 shows another embodiment of a footplate 58 according to the present disclosure. In this embodiment, there is an additional preload in the footplate 58. More particularly, the additional preload derives from a dip in the toe 42 with respect to the toe platform 14, such that the toe 42 makes an angle γ with the toe platform. The dip in the big toe area yields a greater spring force for purposes of footplate 58.

The normal human gait starts at heel strike which is at the back/outside portion of the heel. As gait progresses, the foot rolls through the arch area and the center of gait starts to move medially. In the human gait, the last thing that leaves the ground is the big toe. Therefore, if the big toe is the last thing that leaves the ground, then the big toe area of the footplate must also be the last thing that leaves the ground. To accomplish this objective, the big toe area of the disclosed footplate advantageously dips and provides the last thing on the ground with more associated spring. Having an angle γ gives the footplate 58 an increased spring loading rate. The angle γ may range from about 1° to about 25° in exemplary embodiments of the present disclosure, and is preferably about 15°.

When the footplate 58 is placed on a flat surface, the heel and the toe are the only parts that touch the surface. Therefore, when one applies weight to the footplate 58, then the entire footplate 58 generally flattens, thus preloading the spring effect of the footplate 58. This additional preloading may make a big difference in the functional attributes of the disclosed AFO system. When one flexes his or her foot to walk or run, the spring load is increased, giving the user an extra push.

In use, the footplate of the present disclosure advantageously generally functions such that:
(i) in the absence of an applied force to the top surface of the footplate and with the bottom surface of the footplate resting on a horizontal surface (a) the bottom surface of the toe platform region and the heel region contact the horizontal surface; and (b) the footplate bows upward in the longitudinal arch pad region relative to the toe platform region and the heel region, such that the bottom surface of the longitudinal arch pad region is spaced from the horizontal surface, and
(ii) in response to a force being applied to the top surface of the footplate with the bottom surface of the toe platform region and the heel region in contact with a horizontal surface the bowed longitudinal arch pad region flexes downward relative to the toe pad region and the heel region to load a first pre-load force in the footplate and
(iii) in response to the heel region thereafter moving upward from the horizontal surface while maintaining the toe platform region in contact with the horizontal surface (c) the bowed longitudinal arch pad reaches flexes upward and the first pre-load force is released to deliver a propulsive force to the top surface of the footplate; and (d) the footplate flexes to define a flex angle between the toe platform region and the longitudinal arch pad region to load a second pre-load force into the footplate; and
(iv) in response to the toe platform region thereafter moving upward from and out of contact with the horizontal surface the footplate returns from its flexed position to eliminate the flex angle and the second pre-load force is released to deliver a propulsive force to the top surface of the footplate.

Of note, a secondary benefit to the arched shape of the footplate from heel-to-toe is that when the footplate deflects, the posterior strut moves forward, providing added "push" during gait. As will be readily apparent to persons skilled in the art, the relationship of the downward force and forward force will exist across all disclosed implementations of the AFO products disclosed herein.

Turning to FIGS. 8-15, a series of views of an exemplary AFO 100 are provided. The AFO 100 includes a footplate 102 and a brace structure 104 extending upwardly with respect to the footplate 102. Although exemplary brace structures are disclosed in the present application, the present disclosure is not limited by or to the exemplary brace structures disclosed herein. Rather, any brace structure that is effective to secure the footplate relative to the leg of a user may be employed. Of note, the brace structure may be secured with respect to the user's leg from the front, back, side and/or a combination thereof. Thus, the securement mechanism may be accessed from an anterior, posterior, medial and/or lateral direction relative to the patient's leg.

The footplate 102 generally includes the features and functions of the various footplates described above, and is generally fabricated in like manner. The brace structure 104 generally includes a securing region 106 and an intermediate extension arm 108 that joins the footplate 102 with the securing region 106. In the exemplary embodiment of FIGS. 8-15, the intermediate extension arm 108 advantageously defines an arcuate geometry that extends from a side of the footplate 102 to a central position above the heel region of the footplate 102. In this way, the intermediate extension arm 108 advantageously connects the securing region 106 of the brace structure 104 relative to the footplate 102 without unduly interfering with or abrading the lower leg region of the user.

The securing region 106 generally defines a semi-cylindrical geometry that is configured and dimensioned to cooperate with the user's rear ankle/calf region. Slots or openings 110 are generally defined in the securing region 106 to reduce weight and materials cost, as well as to reduce the potential for discomfort when attached with respect to the user's lower leg. In the exemplary embodiment of FIGS. 8-15, a central spine 112 is defined between opposed slots/openings 110 of securing region 106 to impart structural stability thereto. A strap or other securement member (not pictured) generally cooperates with the securing region 106 and is adapted to extend around the front of the user's ankle/calf region to secure the AFO 100 relative to the user.

Although the exemplary AFO 100 shown in FIGS. 8-15 is of integral design/construction, it is contemplated that the securing region 106 may be detachably mounted with respect to the footplate 102, e.g., by providing a detachment mechanism at the junction of intermediate extension arm 108 and central spine 112. Alternative mechanisms for joining/detaching the footplate and the securing region 106 of brace structure 104 may be employed without departing from the spirit or scope of the present disclosure, as will be readily apparent to persons skilled in the art.

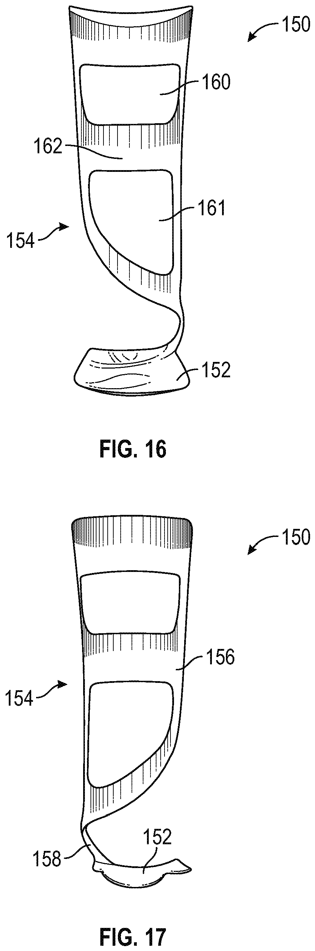

With reference to FIGS. 16-19, an alternative AFO 150 is schematically depicted. The AFO 150 is generally of the same design and operation of AFO 100, including a footplate 152 and a brace structure 154 that includes a securing region 156 and an intermediate extension arm 158 that joins the footplate 152 with the securing region 156. However, unlike AFO 100, the securing region 156 of brace structure 154 does not include a vertically aligned central spine, but instead includes a horizontal member 162 that separates top and bottom slots/openings 160, 161. The overall design of AFO 150 generally facilitates securement with respect to higher points on the lower leg of a user as compared to AFO 100. However, the general features and functions of AFO 150, including the potential for integral and detachable implementations, are the same as compared to AFO 100.

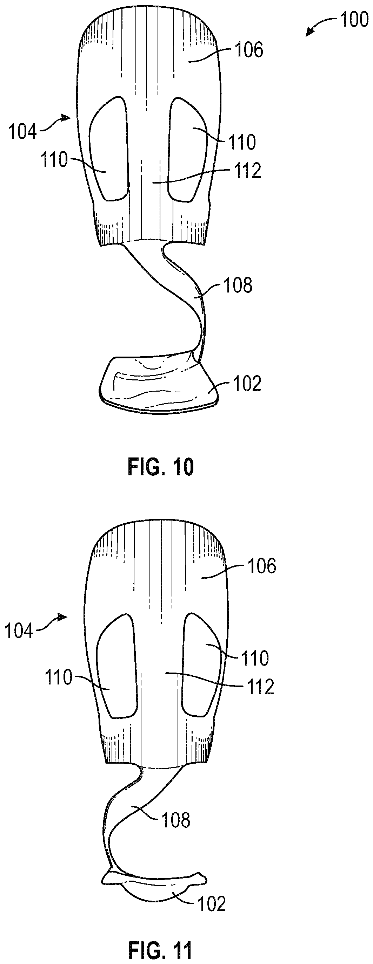

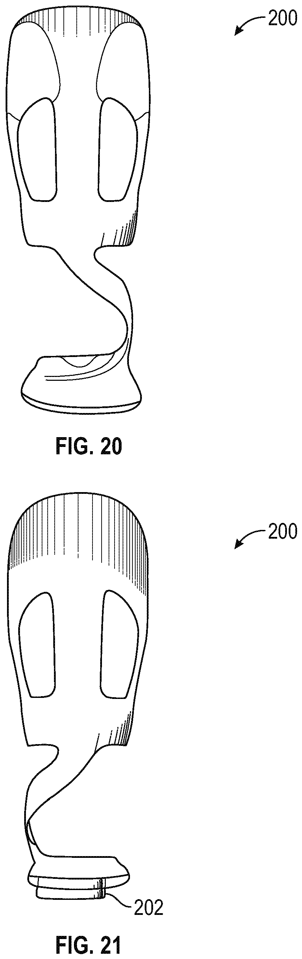

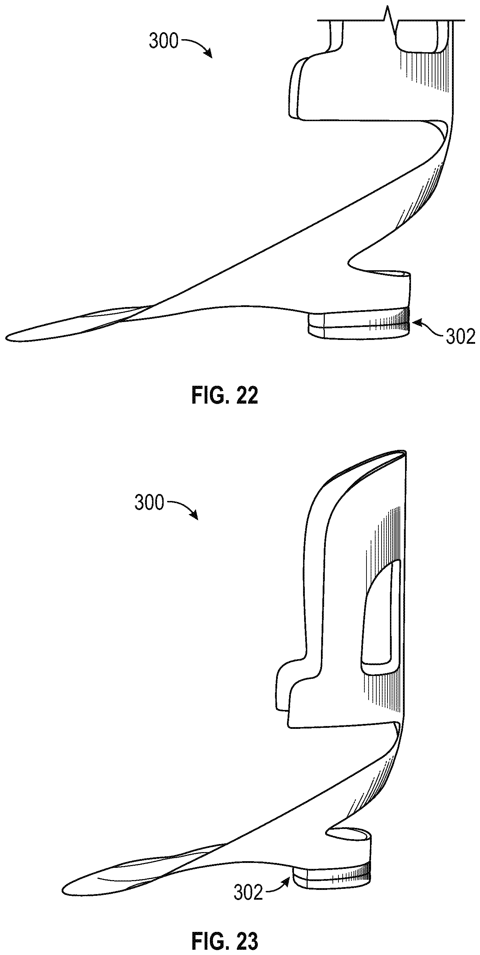

An alternative AFO 200 is schematically depicted in Fig. 20 (front view) and Fig. 21 (rear view). The brace structure of AFO 200 is similar in design to the embodiment shown in Figs. 10-15. The footplate of AFO 200 is noteworthy in that it is associated with a heel structure 202 that may enhance the comfort and/or function thereof. The height of heel structure 202 may be selected based on comfort and/or functional considerations, as will be apparent to persons skilled in the art. Figs. 22-24 provide schematic side views of exemplary AFO 300 that further highlight an embodiment of the present disclosure that include a heel structure 302. Of note, by ensuring appropriate geometric and structural characteristics of the footplates associated with AFO's 200, 300, the advantageous propulsive properties of the disclosed AFO's are not impacted by the inclusion of a heel structure according to the present disclosure.

Of note, in exemplary embodiments of the disclosed AFO, the footplate and the brace structure continuously define inner and outer surfaces of the orthosis, and combine to form a monolithic structure. Moreover, the disclosed AFO may be fabricated with variable thicknesses, e.g., in the region of the brace support, and an uninterrupted variable thicknesses may be defined by the footplate and the brace structure. In fabricating the disclosed AFO, it may be desirable to fabricate the brace structure at least in part from fibers, e.g., pre-preg carbon fibers, and to interleave the brace structure fibers with layers of the footplate fibers so as to join the respective structures, e.g., during the molding process. Of further note, it is contemplated that the junction between the footplate and the brace structure may accommodate relative movement therebetween, e.g., a relative sliding movement, so as to facilitate comfort and/or therapeutic results. Thus, for example, a pin-in-track design may be employed to employ relative movement between the noted components.

The disclosed AFO has many advantages. The AFO may be specifically designed for different ailments/maladies and may be designed to deliver different levels of propulsive force, thereby enhancing the recuperative process. The disclosed AFO may provide more "spring" or "push" to an individual closer to full recovery, while providing less spring/push to users who are less ambulatory. The footplate portion of the disclosed AFO may replace the insole that comes with off the shelf footwear, although alternative modes of combining the disclosed AFO with a user's footwear needs and options may be employed, as will be readily apparent to persons skilled in the art. The footplate associated with the disclosed AFO advantageously pre-loads a propulsive force while the user is simply standing and this spring effect is amplified when the toes are dorsiflexed (turned up). As the foot leaves the ground, preparing for its next heel strike, the footplate unloads into plantarflexion at a rapid rate using ground reactive force to propel the user forward by amplifying push-off.

While the disclosure has been described with reference to several embodiments, it will be understood by those skilled in the art that various changes may be made and equivalents may be substituted for elements thereof without departing from the scope of the disclosure. In addition, many modifications may be made to adapt a particular situation or material to the teachings of the disclosure without departing from the essential scope thereof. Therefore, it is intended that the disclosure not be limited to the particular embodiments disclosed as the best mode contemplated for carrying out this disclosure, but that the disclosure will include all embodiments falling within the scope of the appended claims.

## Claims

1. A flexible member for use in connection with or as an orthotic, an orthotic insert or an ankle foot orthosis, comprising:
a structure (1000; 10; 30; 58; 102; 152) defining (i) a toe platform region (14), (ii) a longitudinal arch pad region (18), and (iii) a heel region (22), and the structure further defining (i) a top surface, (ii) a bottom surface, and (iii) a center axis; and
wherein the structure includes a plurality of fiber layers (1002A, 1002B, 1002C) of varying lengths,
wherein each of the fiber layers includes a plurality of unidirectionally aligned fibers that are angled at between about 10° and 20° relative to the center axis such that the plurality of unidirectionally aligned fibers are angled medially-to-laterally from the heel region to the toe platform region,
wherein the structure is arched from the heel region to the toe platform region;
wherein the toe platform region defines a medial distal aspect and a lateral aspect, and
wherein the structure is torqued so that medial distal aspect is lower than the lateral aspect of the toe platform region.

2. The flexible member according to claim 1, wherein the plurality of unidirectionally aligned fibers are carbon fibers.

3. The flexible member according to claim 1, wherein the flexible member is incorporated into an ankle foot orthosis (100; 150; 200; 300) that includes a footplate (102; 152) and a brace structure (104; 154), and wherein the brace structure includes a securing region (106; 156) and an intermediate extension arm (108; 158) that joins the securing region (106; 156) with respect to the footplate (102; 152).

4. The flexible member according to claim 43, wherein the brace structure is detachably mounted with respect to the footplate.

5. The flexible member according to claim 1, wherein the structure is configured and dimensioned such that:
a. in the absence of an applied force to the top surface of the structure and with the bottom surface of the structure resting on a horizontal surface: (i) the bottom surface of the toe platform region and the heel region contact the horizontal surface; and (ii) the structure bows upward in the longitudinal arch pad region relative to the toe platform region and the heel region such that the bottom surface of the longitudinal arch pad region is spaced from the horizontal surface, and
b. in response to a force being applied to the top surface of the structure with the bottom surface of the toe platform region and the heel region in contact with a horizontal surface, the bowed longitudinal arch pad region flexes downward relative to the toe pad region and the heel region to load a first pre-load force in the structure; and
c. in response to the heel region thereafter moving upward from the horizontal surface while maintaining the toe platform region in contact with the horizontal surface: (i) the bowed longitudinal arch pad flexes upward and the first pre-load force is released to deliver a propulsive force to the top surface of the structure; and (ii) the structure flexes to define a flex angle between the toe platform region and the longitudinal arch pad region to load a second pre-load force into the structure; and
d. in response to the toe platform region thereafter moving upward from and out of contact with the horizontal surface, the structure returns from its flexed position to eliminate the flex angle and the second pre-load force is released to deliver a propulsive force to the top surface of the structure.

6. The flexible member according to claim 3, wherein the footplate and the brace structure continuously define inner and outer surfaces that combine to form a monolithic structure.

7. The flexible member according to claim 3, wherein an uninterrupted variable thickness is defined by the footplate and the brace structure.

8. The flexible member according to claim 3, wherein the brace structure and at least two fiber layers of the footplate are integrated such that the fiber layers of the footplate are interleaved with fibers associated with the brace structure.

9. The flexible member according to claim 1, further comprising a heel structure associated with the bottom surface of the structure in the heel region.

10. The flexible member according to claim 1, wherein the plurality of unidirectionally aligned fibers that are angled at between about 12° and 18° relative to the center axis such that the plurality of unidirectionally aligned fibers are angled medially from the heel region to the toe platform region.

11. The flexible member according to claim 1, wherein the plurality of unidirectionally aligned fibers that are angled at about 15° relative to the center axis such that the plurality of unidirectionally aligned fibers are angled medially from the heel region to the toe platform region.

## Patentansprüche

1. Ein flexibles Teil zur Verwendung in Verbindung mit oder als eine Orthese, eine orthesische Einlage oder eine Fußgelenkorthese aufweisend:
ein Gebilde (1000; 10; 30; 58; 102; 152), das (i) einen Zehenplattformbereich (14), (ii) einen longitudinalen Gewölbestützenbereich (18), und (iii) einen Fersenbereich (22) definiert, und das Gebilde ferner (i) eine Oberseite, (ii) eine Bodenfläche, und (iii) eine Mittelachse definiert; und
wobei das Gebilde eine Vielzahl von Faserschichten (1002A, 1002B, 1002C) mit unterschiedlichen Längen umfasst,
wobei jede der Faserschichten eine Vielzahl von in einer Richtung ausgerichteten Fasern umfasst, die mit der Mittelachse einen Winkel von etwa 10° bis 20° derart einschließen, dass die Vielzahl der in einer Richtung ausgerichteten Fasern medial bis lateral von dem Fersenbereich zu dem Zehenplattformbereich schräg verlaufen,
wobei das Gebilde zwischen dem Fersenbereich und dem Zehenplattformbereich bogenförmig ist;
wobei der Zehenplattformbereich einen medialen distalen Aspekt und einen lateralen Aspekt definiert,
und
wobei das Gebilde angezogen ist, so dass der mediale distale Aspekt kleiner als der laterale Aspekt des Zehenplattformbereichs ist.

2. Das flexible Teil nach Anspruch 1, wobei die Vielzahl der in einer Richtung ausgerichteten Fasern Karbonfasern sind.

3. Das flexible Teil nach Anspruch 1, wobei das flexible Teil in eine Fußgelenkorthese (100; 150; 200; 300) eingesetzt ist, die eine Fußplatte (102; 152) und ein Versteifungssgebilde (104; 154) umfasst, und wobei das Versteifungssgebilde einen Sicherungsbereich (106; 156) und einen dazwischenliegenden Verlängerungsarm (108; 158) umfasst, der den Sicherungsbereich (106; 156) mit der Fußplatte (102; 152) verbindet.

4. Das flexible Teil nach Anspruch 3, wobei das Versteifungssgebilde abnehmbar in Bezug auf die Fußplatte befestigt ist.

5. Das flexible Teil nach Anspruch 1, wobei das Gebilde derart ausgebildet und dimensioniert ist, dass
a. in Abwesenheit einer auf die Oberseite des Gebildes wirkenden Kraft und mit der Bodenfläche des Gebildes auf einer horizontalen Ebene ruhend: (i) die Bodenfläche des Zehenplattformbereichs und des Fersenbereichs die horizontale Fläche kontaktieren; und (ii) das Gebilde sich derart in den longitudinalen Gewölbestützenbereich in Bezug auf den Zehenplattformbereich und den Fersenbereich nach oben wölbt, dass die Bodenfläche des longitudinalen Gewölbestützenbereich zu der horizontalen Fläche im Abstand angeordnet ist, und
b. als Antwort auf eine auf die Oberseite des Gebildes wirkenden Kraft mit der Bodenfläche des Zehenplattformbereich und des Fersenbereichs mit der horizontalen Fläche in Kontakt befindend sich der bogenförmige longitudinale Gewölbestützenbereich in Bezug auf den Zehenplattformbereich und den Fersenbereich nach unten durchbiegt, so dass eine erste Vorspannkraft in das Gebilde eingebracht wird; und
c. als Antwort auf den sich danach von der horizontalen Fläche nach oben bewegenden Fersenbereich, während der Kontakt des Zehenplattformbereichs mit der horizontalen Fläche aufrechterhalten wird: (i) der bogenförmige longitudinale Gewölbestützenbereich sich nach oben biegt und die erste Vorspannkraft freigegeben wird, um eine Antriebskraft auf die Oberseite des Gebildes auszuüben; und (ii) das Gebilde sich durchbiegt, um zwischen dem Zehenplattformbereich und dem longitudinalen Gewölbestützenbereich einen Durchbiegungswinkel zu definieren, um eine zweite Vorspannkraft in das Gebilde einzubringen; und
d. als Antwort auf den sich danach von der horizontalen Fläche nach oben von und aus dem Kontakt mit der horizontalen Fläche bewegenden Zehenplattformbereich das Gebilde wieder aus der Durchbiegungsposition zurückkehrt, um den Durchbiegungswinkel zu eliminieren, und die zweite Vorspannkraft wird freigegeben, um eine Antriebskraft auf die Oberseite des Gebildes aufzubringen.

6. Das flexible Teil nach Anspruch 3, wobei die Fußplatte und das Versteifungsgebilde kontinuierlich innere und äußere Flächen definieren, die sich zur Ausbildung einer monolithischen Struktur vereinen.

7. Das flexible Teil nach Anspruch 3, wobei durch die Fußplatte und das Versteifungsgebilde eine ununterbrochene variable Dicke definiert wird.

8. Das flexible Teil nach Anspruch 3, wobei das Versteifungsgebilde und wenigstens zwei Faserschichten der Fußplatte derart integriert sind, dass die Faserschichten der Fußplatte mit den Fasern, die mit dem Versteifungsgebilde zusammenhängen, verschachtelt sind.

9. Das flexible Teil nach Anspruch 1, ferner ein Fersengebilde aufweisend, das mit der Bodenfläche des Gebildes in dem Fersenbereich zusammenhängt.

10. Das flexible Teil nach Anspruch 1, wobei die Vielzahl der in einer Richtung ausgerichteten Fasern einen Winkel mit der Mittelachse von etwa 12° bis 18° derart einschließen, dass die Vielzahl der in einer Richtung ausgerichteten Fasern von dem Fersenbereich zu dem Zehenplattformbereich medial schräg verlaufen.

11. Das flexible Teil nach Anspruch 1, wobei die Vielzahl der in einer Richtung ausgerichteten Fasern einen Winkel mit der Mittelachse von etwa 15° derart einschließen, dass die Vielzahl der in einer Richtung ausgerichteten Fasern von dem Fersenbereich zu dem Zehenplattformbereich medial schräg verlaufen.

## Revendications

1. Élément flexible destiné à être utilisé en lien avec ou comme une orthèse, un insert orthopédique ou une orthèse pied/cheville, comprenant :
une structure (1000; 10; 30; 58; 102; 152) définissant (i) une zone de plateforme d'orteils (14), (ii) une zone de soutien de voûte plantaire longitudinale (18), et (iii) une zone de talon (22), et la structure définissant en outre (i) une surface supérieure, (ii) une surface inférieure, et (iii) un axe central; et
dans lequel la structure comprend une pluralité de couches de fibres (1002A, 1002B, 1002C) de longueurs variables,
dans lequel chacune des couches de fibres comprend une pluralité de fibres alignées unidirectionnellement qui sont inclinées entre environ 10° et 20° par rapport à l'axe central, de telle sorte que la pluralité de fibres alignées unidirectionnellement sont inclinées de façon médiane à latérale à partir de la zone de talon jusqu'à la zone de la plateforme d'orteils,
dans lequel la structure est arquée à partir de la zone de talon jusqu'à la zone de la plateforme d'orteils;
dans lequel la zone de plateforme d'orteils définit un aspect distal médial et un aspect latéral, et
dans lequel la structure est serrée de sorte que l'aspect distal médial est inférieur à l'aspect latéral de la zone de plateforme d'orteils.

2. Élément flexible selon la revendication 1, dans lequel la pluralité de fibres alignées unidirectionnellement sont des fibres de carbone.

3. Élément flexible selon la revendication 1, dans lequel l'élément flexible est incorporé dans une orthèse pied/cheville (100; 150; 200; 300) qui comprend une semelle (102; 152) et une structure de renfort (104; 154), et dans lequel la structure de renfort comprend une zone de fixation (106; 156) et un bras d'extension intermédiaire (108; 158) qui rejoint la zone de fixation (106; 156) par rapport à la semelle (102; 152).

4. Élément flexible selon la revendication 3, dans lequel la structure de renfort est montée de manière amovible par rapport à la semelle.

5. Élément flexible selon la revendication 1, dans lequel la structure est configurée et dimensionnée de telle sorte que :
a. en l'absence d'une force appliquée sur la surface supérieure de la structure et en ayant la surface inférieure de la structure reposant sur une surface horizontale: (i) la surface inférieure de la zone de plateforme d'orteils et la zone de talon entrent en contact avec la surface horizontale; et (ii) la structure s'incline vers le haut dans la zone de soutien de voûte plantaire longitudinale par rapport à la zone de plateforme d'orteils et la zone de talon de telle sorte que la surface inférieure de la zone de soutien de voûte plantaire longitudinale soit espacée de la surface horizontale,
b. en réponse à une force appliquée à la surface supérieure de la structure en ayant la surface inférieure de la zone de la plateforme d'orteils et la zone de talon en contact avec une surface horizontale, la zone de soutien de voûte plantaire longitudinale inclinée fléchit vers le bas par rapport à la zone de soutien d'orteils et à la zone de talon pour charger une première force de précharge dans la structure ; et
c. en réponse à la zone de talon se déplaçant ensuite vers le haut à partir de la surface horizontale tout en maintenant la zone de la plateforme d'orteils en contact avec la surface horizontale: (i) le soutien de voûte plantaire longitudinal incliné fléchit vers le haut et la première force de précharge est relâchée pour fournir une force de propulsion à la surface supérieure de la structure; et (ii) la structure fléchit pour définir un angle de flexion entre la zone de plateforme d'orteils et la zone de soutien de voûte plantaire longitudinale pour charger une deuxième force de précharge dans la structure; et
d. en réponse à la zone de plateforme d'orteils se déplaçant ensuite vers le haut depuis et hors du contact avec la surface horizontale, la structure revient de sa position fléchie pour éliminer l'angle de flexion et la deuxième force de précharge est libérée pour fournir une force de propulsion à la surface supérieure de la structure.

6. Élément flexible selon la revendication 3, dans lequel la semelle et la structure de renfort définissent en continu des surfaces intérieure et extérieure qui se combinent pour former une structure monolithique.

7. Élément flexible selon la revendication 3, dans lequel une épaisseur variable ininterrompue est définie par la semelle et la structure de renfort.

8. Élément flexible selon la revendication 3, dans lequel la structure de renfort et au moins deux couches de fibres de la semelle sont intégrées de telle sorte que les couches de fibres de la semelle sont entrelacées avec des fibres associées à la structure de renfort.

9. Élément flexible selon la revendication 1, comprenant en outre une structure de talon associée à la surface inférieure de la structure dans la zone de talon.

10. Élément flexible selon la revendication 1, dans lequel la pluralité de fibres alignées unidirectionnellement qui sont inclinées entre environ 12° et 18° par rapport à l'axe central de sorte que la pluralité de fibres alignées unidirectionnellement sont inclinées médialement de la zone de talon à la zone de plateforme d'orteils.

11. Élément flexible selon la revendication 1, dans lequel la pluralité de fibres alignées unidirectionnellement qui sont inclinées à environ 15° par rapport à l'axe central de telle sorte que la pluralité de fibres alignées unidirectionnellement sont inclinées médialement de la zone de talon à la zone de plateforme d'orteils.
